# EUROPÄISCHE PATENTSCHRIFT

(11) **EP 3 515 519 B1**
(45) Veröffentlichungstag und Bekanntmachung des Hinweises auf die Patenterteilung: **01.11.2023**
(21) Anmeldenummer: 17769056.7
(22) Anmeldetag: 18.09.2017
(51) Int. Cl.: A61M 1/00

(54) **VORRICHTUNG ZUM ABSAUGEN VON KÖRPERFLUIDEN UND ZUM ZUFÜHREN EINER SUBSTANZ**
DEVICE FOR SIPHONING BODILY FLUIDS AND FOR DELIVERING A SUBSTANCE
DISPOSITIF D'ASPIRATION DE FLUIDES CORPORELS ET D'ACHEMINEMENT D'UNE SUBSTANCE

(30) Priorität: 20.09.2016 EP 16189671
(43) Veröffentlichungstag der Anmeldung: 31.07.2019
(60) Teilanmeldung: 19199463.1
(73) Patentinhaber: Medela Holding AG, 6340 Baar (CH)
(72) Erfinder: EHLERT, Hilmar, 6052 Hergiswil (CH)
(74) Vertreter: Grünecker Patent- und Rechtsanwälte PartG mbB
(86) Internationale Anmeldenummer: PCT/EP2017/073466
(87) Internationale Veröffentlichungsnummer: WO 2018/054834

(56) Entgegenhaltungen:
- DE-U1-202015 006 341
- FR-A1- 2 960 423
- US-A1- 2006 025 727
- US-A1- 2013 085 462
- US-B1- 6 454 543

## Beschreibung

### TECHNISCHES GEBIET

Die vorliegende Erfindung betrifft eine Vorrichtung zum Absaugen von Körperfluiden und zum Zuführen einer Substanz zu einem menschlichen oder tierischen Körper. Derartige Vorrichtungen werden insbesondere im medizinischen Bereich verwendet, beispielsweise in der mit Instillation oder Irrigation kombinierten Unterdruck-Wundtherapie, in der Augenchirurgie oder bei der Fettabsaugung.

### STAND DER TECHNIK

Im medizinischen Bereich gibt es vielfältige Anwendungen, bei denen einerseits Körperfluide oder Sekrete aus Körperkavitäten oder Wunden mittels einer Pumpe abgesaugt und andererseits eine Substanz dem Körper zugeführt werden. Mögliche Anwendungsgebiete betreffen insbesondere die mit Instillation kombinierte Unterdruck-Wundtherapie, die Augenchirurgie und die Liposuktion (Fettabsaugung). Je nach Anwendung erfolgen die Absaugung und die Zuführung dabei gleichzeitig, nacheinander und/oder abwechslungsweise intermittierend.

Bei der zuzuführenden Substanz kann es sich beispielsweise um eine physiologische oder nicht physiologische Kochsalzlösung, ein Arzneimittel oder um ein Gemisch davon handeln. Die Substanz kann zum Beispiel zur Förderung der Wundheilung, zur Verhinderung von Infektionen oder zur lokalen Anästhesie dienen. Das Zuführen der Substanz kann somit zur Spülung oder therapeutischen, diagnostischen und/oder präventiven Zwecken dienen.

Oft wird zum Zuführen der Substanz ähnlich wie bei der herkömmlichen Infusion ein mit der zuzuführenden Substanz gefüllter Flüssigkeitsbeutel oder eine Flasche erhöht über der zu behandelnden Körperstelle angeordnet, so dass die Substanz aufgrund des hydrostatischen Druckes durch eine Zuführleitung der zu behandelnden Stelle zugeführt wird. Separat dazu werden die Körperfluide von einer Vakuumpumpe via einer entsprechenden Leitung abgesaugt.

Um eine bessere Einstellung und Regelung beim Zuführen der Substanz zu ermöglichen, und/oder um unabhängig von der Anordnung und insbesondere der Höhenlage des mit der Substanz gefüllten Flüssigkeitsbehälters zu sein, sind auch Systeme hinlänglich bekannt, bei denen die Zuführung der Substanz zum Körper mittels einer Pumpe, insbesondere einer sog. Peristaltik- oder Schlauchpumpe erfolgt.

Beispielsweise zeigt die WO 2016/054470 eine derartige Vorrichtung mit einer ersten Pumpe zum Zuführen von Substanzen zu einem Wundbereich und mit einer zweiten Pumpe zum Absaugen von Fluiden aus dem Wundbereich.

Die Bedienung derartiger Vorrichtungen ist für den Anwender oft umständlich, da einerseits die Pumpe zum Zuführen der Substanzen und andererseits die Pumpe zum Absaugen korrekt installiert und eingestellt werden müssen.

Um den apparativen Aufwand bei der Behandlung zu verringern, sind Geräte bekannt, bei denen die Pumpe für das Absaugen der Körperfluide sowie die Pumpe für das Zuführen der Substanz in einem gemeinsamen Gehäuse untergebracht sind.

Beispielsweise offenbart die WO 2015/091070 ebenso wie die US 2008/0154184, die US 2008/0154182, die US 8,591,453 und die US 2008/0154185 ein Gerät mit zwei in einem gemeinsamen Gehäuse angeordneten Pumpen, von denen eine zum Absaugen von Körperfluiden und die andere zum Zuführen einer Substanz dient.

Auch in der US 2014/0163487 ist ein derartiges Gerät mit zwei Pumpen offenbart, wobei hier der Pumpenkopf einer Peristaltikpumpe, welche zum Zuführen einer Substanz zum Körper dient, auf der Aussenseite des Pumpaggregatgehäuses angeordnet ist. Ein Flüssigkeitsbehälter, welcher zur Aufnahme einer Instillationsflüssigkeit dient, ist an das Pumpaggregatgehäuse anschliessbar. Am Flüssigkeitsbehälter ist eine Schlauchführung ausgebildet, aufgrund welcher der Pumpenkopf, wenn der Behälter am Pumpaggregatgehäuse angeschlossen ist, eine entsprechende Pumpwirkung auf den aus dem Behälterinneren herausführenden Instillationsschlauch ausübt, um so die Instillationsflüssigkeit zum Körper hin zu pumpen.

Diese Vorrichtungen, welche einerseits zum Absaugen von Körperfluiden sowie andererseits zum Zuführen einer Substanz dienen, haben meist ein beträchtliches Volumen sowie Gewicht. Ausserdem sind sie aufwändig und entsprechend kostenintensiv in der Herstellung.

Weitere gattungsgemässe Vorrichtungen sind zum Beispiel in der EP 2 883 555, der CN 104771801, der US 4,634,024 offenbart.

US 2006/025727 A1, US 2013/085462 A1, DE 20 2015 006341 U1 und FR 2 960 423 A1 betreffen jeweils gattungsgemäßen Stand der Technik.

### DARSTELLUNG DER ERFINDUNG

Es ist deshalb eine Aufgabe der Erfindung, eine vielseitig einsetzbare Vorrichtung zum Absaugen von Körperfluiden und zum Zuführen einer Substanz zu einem menschlichen oder tierischen Körper zu schaffen, welche ein geringes Volumen und Gewicht aufweist. Die Vorrichtung sollte ausserdem für den Anwender einfach handhabbar sein.

Zur Lösung dieser Aufgabe wird eine Vorrichtung vorgeschlagen, wie sie in Anspruch 1 angegeben ist. Vorteilhafte Ausgestaltungen der Erfindung sind in den abhängigen Ansprüchen angegeben. Die vorliegende Erfindung stellt also eine Vorrichtung zum Absaugen von Körperfluiden und zum Zuführen einer Substanz zu einem menschlichen oder tierischen Körper zur Verfügung, aufweisend
eine erste Pumpe zum Absaugen der Körperfluide;
eine zweite Pumpe, um mittels der zweiten Pumpe die Substanz zum Körper zu befördern; sowie
einen Antrieb zum Antreiben der ersten Pumpe; wobei derselbe Antrieb, welcher zum Antreiben der ersten Pumpe dient, auch zum Antreiben der zweiten Pumpe dient,
dadurch gekennzeichnet, dass die Vorrichtung ausserdem zumindest einen ersten Freilauf aufweist, mittels welchem die erste Pumpe oder die zweite Pumpe an den Antrieb gekoppelt ist, wobei es sich bei der ersten Pumpe um eine Vakuumpumpe handelt, und wobei die Vorrichtung zusätzlich ein Ventil aufweist, mittels welchem die Vakuumpumpe mit der Umgebung verbindbar ist, um zumindest teilweise anstatt Körperfluide Luft aus der Umgebung anzusaugen.

Indem derselbe Antrieb zum Antreiben, insbesondere zum gleichzeitigen Antreiben, von beiden Pumpen dient, kann die Vorrichtung insgesamt kleiner dimensioniert werden und zudem ein geringeres Gewicht aufweisen. Die Vorrichtung kann dadurch insbesondere derart ausgebildet werden, dass sie tragbar ist, das heisst, dass sie von einem Benutzer alleine und ohne übermässigen Kraftaufwand bequem getragen werden kann. Vorteilhaft weist die Vorrichtung zudem eine insgesamt kompakte Bauweise auf. Aufgrund des nur einen Antriebs ist auch die Fehleranfälligkeit verringert, und die Vorrichtung ist insgesamt kostengünstiger herstellbar.

Bei der ersten Pumpe handelt es sich erfindungsgemäß um eine Vakuumpumpe, insbesondere um eine Membranpumpe. Eine Membranpumpe weist in der Regel zumindest eine Membran sowie eine von dieser begrenzte Pumpkammer auf. Die Vorrichtung weist zusätzlich ein Ventil, insbesondere ein Pneumatikventil, auf, mittels welchem die Vakuumpumpe mit der Umgebung verbindbar ist, um zumindest teilweise oder sogar vollständig anstatt Körperfluide Luft aus der Umgebung anzusaugen. Das Ventil kann insbesondere mit einem Vakuumanschluss der Vakuumpumpe verbunden sein, an welchen eine Saugleitung anschliessbar ist. Mittels Umstellen des Ventils kann der Vakuumanschluss bei Bedarf zumindest teilweise oder sogar vollständig statt mit der Saugleitung mit der Umgebung verbunden werden. Das Ventil ermöglicht es, die Saugleistung zum Absaugen der Körperfluide bei gleichbleibender Motorleistung zu variieren.

Bei der zweiten Pumpe handelt es sich bevorzugt um eine Peristaltikpumpe. Peristaltikpumpen sind auch unter dem Begriff Schlauchpumpen bekannt und eignen sich besonders gut für eine pulsierende Zuführung einer Substanz, insbesondere einer fluiden Substanz wie einer Flüssigkeit, zum Körper. Eine Peristaltikpumpe weist in der Regel zumindest einen drehbar gelagerten Pumpenkopf auf, an welchem zum Beispiel Anpressrollen oder Gleitschuhe angebracht sind.

Die erfindungsgemässe Vorrichtung wird für medizinische Zwecke eingesetzt, insbesondere zu der mit Instillation oder Irrigation kombinierten Unterdruckbehandlung von Wunden am menschlichen oder tierischen Körper. Andere Anwendungsgebiete sind jedoch möglich, beispielsweise die kombinierte Fettabsaugung und Spülung bei der Liposuktion oder das Spülen von Kathetern zur Vermeidung von Verstopfungen oder die kombinierte Absaugung und Spülung bei der Augenchirurgie. Der Freilauf kann insbesondere bewirken, dass je nach Drehrichtung des Antriebs entweder beide Pumpen zusammen angetrieben werden, oder dass nur die erste Pumpe bzw. nur die zweite Pumpe angetrieben wird. Dies kann für bestimmte Anwendungen erwünscht sein.

Der erste Freilauf kann zum Beispiel die erste Pumpe an den Antrieb koppeln. Vorzugsweise ist dann ein zweiter Freilauf vorhanden, mittels welchem die zweite Pumpe entweder beide Pumpen zusammen angetrieben werden, oder dass nur die erste Pumpe bzw. nur die zweite Pumpe angetrieben wird. Dies kann für bestimmte Anwendungen erwünscht sein.

Der erste Freilauf kann zum Beispiel die erste Pumpe an den Antrieb koppeln. Vorzugsweise ist dann ein zweiter Freilauf vorhanden, mittels welchem die zweite Pumpe an den Antrieb gekoppelt ist. Der zweite Freilauf weist vorteilhaft eine im Vergleich zum ersten Freilauf entgegengesetzte Freilaufrichtung auf. Es kann dadurch insbesondere sichergestellt werden, dass je nach Drehrichtung des Antriebs entweder die erste Pumpe oder die zweite Pumpe angetrieben wird, nicht jedoch beide zusammen. Das heisst, entweder wird eine Substanz zum Körper zugeführt, oder es werden Körperflüssigkeiten abgesaugt, jedoch nicht beides gleichzeitig.

Beim ersten und, falls vorhanden, zweiten Freilauf kann es sich beispielsweise um einen Klemmkörper-Freilauf, um eine Schlingfederkupplung (Federwickel-Freilauf) oder um eine selbstsynchronisierende Schaltkupplung handeln.

Beim Antrieb handelt es sich in der Regel um einen Motor, insbesondere um einen Elektromotor. In einer insbesondere bevorzugten Ausführungsform handelt es sich um einen bürstenlosen Gleichstrommotor, da ein solcher in der Regel bei tiefen Drehzahlen von weniger als 100U/min betrieben werden kann. Ein bürstenloser Gleichstrommotor lässt zudem eine Druckregelung mit relativ kleiner Amplitude zu, wodurch eine sehr genaue Steuerung des Druckes (Unterdruck oder Überdruck) möglich wird.

Die Vorrichtung weist vorteilhaft ein Pumpaggregatgehäuse auf mit einem Innenraum, in welchem zumindest die erste Pumpe und der Antrieb untergebracht sind. Die zweite Pumpe kann ebenfalls im Innenraum des Pumpaggregatgehäuses angeordnet sein. Vorzugsweise ist jedoch entweder die zweite Pumpe auf einer Aussenseite des Pumpaggregatgehäuses angeordnet.

Eine einfache Ausführungsform ergibt sich insbesondere dann, wenn die Vorrichtung einen Antriebsstrang aufweist, bei welchem der Antrieb zwischen der ersten Pumpe und der zweiten Pumpe angeordnet ist.

Um eine besonders vielseitige Einsetzbarkeit der Vorrichtung zu ermöglichen, ist diese vorteilhaft derart ausgebildet, dass die Pumpleistung der ersten Pumpe einerseits und die Pumpleistung der zweiten Pumpe andererseits unabhängig voneinander einstellbar sind. Die Einstellbarkeit bezieht sich hier weniger auf die Herstellung, als vielmehr auf die Verwendung der fertig hergestellten Vorrichtung. Die unabhängige Einstellbarkeit der Pumpleistungen der ersten und der zweiten Pumpe kann auf verschiedene Arten und Weisen erreicht werden. So kann dies zum Beispiel mittels einem oder mehreren Freiläufen in Kombination mit einem an einer Vakuumpumpe angeschlossenem Ventil erreicht werden. Auch die Verwendung von Getrieben in Kombination mit zum Beispiel einem Freilauf ist möglich. Weitere Möglichkeiten sind denkbar.

In bestimmten Ausführungsformen kann somit ein Antriebsstrang mit zumindest einem Getriebe vorgesehen sein, mittels welchem der Antrieb an die ersten Pumpe oder an die zweite Pumpe gekoppelt ist. Die erste bzw. zweite Pumpe kann aufgrund des Getriebes mit einer im Vergleich zum Antrieb unterschiedlichen Drehzahl angetrieben werden.

In einer bestimmten Ausführungsform ist ein Behälter mit der zuzuführenden Substanz an die Vorrichtung anbringbar. Der Behälter kann ein Identifikationsmerkmal und die Vorrichtung eine Identifikationseinheit aufweisen, um zu identifizieren, welche Art einer Substanz im Behälter enthalten ist. Die Vorrichtung kann dann insbesondere dazu ausgebildet sein, in Abhängigkeit der identifizierten Art der Substanz einen von mehreren möglichen Betriebsmodi zum Antreiben der ersten Pumpe und der zweiten Pumpe auszuwählen oder vorauszuwählen.

### KURZE BESCHREIBUNG DER ZEICHNUNGEN

In den Zeichnungen zeigen:
- Fig. 1: eine perspektivische Ansicht einer schematisch gezeigten Vorrichtung gemäss einer ersten erfindungsgemässen Ausführungsform;
- Fig. 2: eine perspektivische Ansicht der Vorrichtung der Fig. 1, verbunden mit einem Flüssigkeitsbehälter mit Instillationsflüssigkeit;
- Fig. 3: eine perspektivische Ansicht der Vorrichtung der Fig. 1, wobei das Pumpaggregatgehäuse nur mit gestrichelten Linien angedeutet ist;
- Fig. 4: eine Draufsicht auf den Antriebsstrang, den Pumpenkopf der peristaltischen Pumpe und die Membranpumpe der Vorrichtung der Fig. 1;
- Fig. 5: eine perspektivische Ansicht einer schematisch gezeigten Vorrichtung gemäss einer zweiten erfindungsgemässen Ausführungsform, wobei das Pumpaggregatgehäuse nur mit gestrichelten Linien angedeutet ist;
- Fig. 6: eine Draufsicht auf den Antriebsstrang, den Pumpenkopf der peristaltischen Pumpe, die Membranpumpe und das Pneumatikventil der Vorrichtung der Fig. 1;
- Fig. 7: eine perspektivische Ansicht einer schematisch gezeigten Vorrichtung gemäss einer nicht erfindungsgemässen Ausführungsform, wobei die Vorderwand des Pumpaggregatgehäuses weggelassen ist;
- Fig. 8: eine erste perspektivische Ansicht eines Fluidsammelbehälters, der zum Anschliessen an die Vorrichtung der Fig. 7 geeignet ist; sowie
- Fig. 9: eine zweite perspektivische Ansicht des Fluidsammelbehälters der Fig. 8.

### BESCHREIBUNG BEVORZUGTER AUSFÜHRUNGSFORMEN

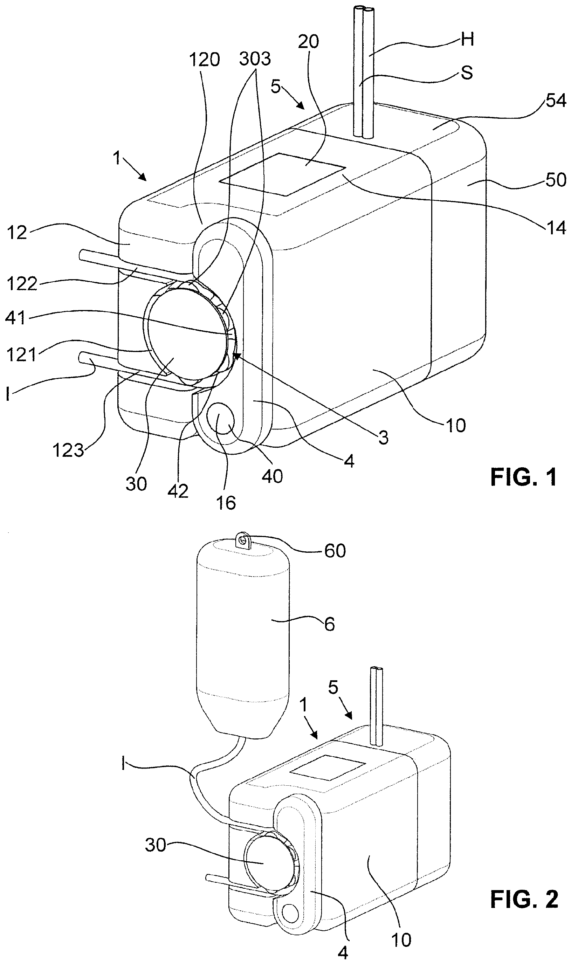

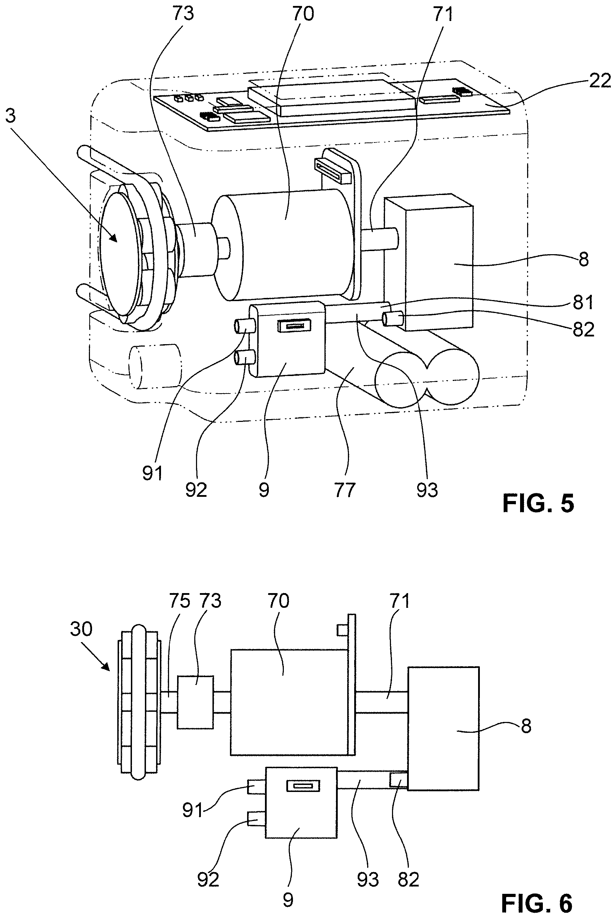

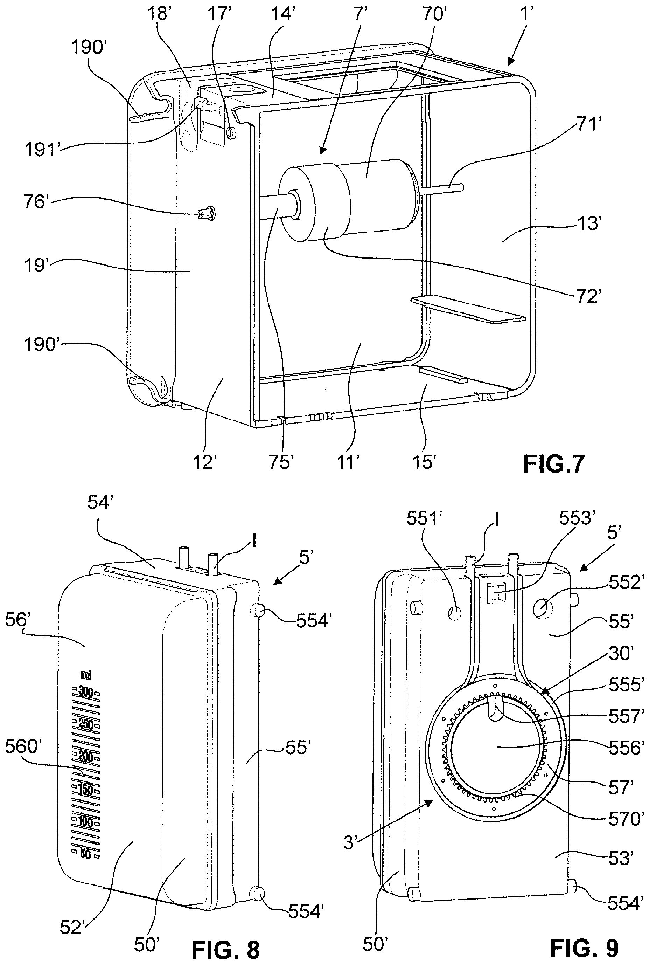

In den Figuren 1 bis 7 sind unterschiedliche Ausführungsformen von Vorrichtungen gezeigt, wobei sich die Figuren 1 bis 4 auf eine erste erfindungsgemässe Ausführungsform, die Figuren 5 und 6 auf eine zweite erfindungsgemässe Ausführungsform und die Figur 7 auf eine dritte nicht erfindungsgemässe Ausführungsform beziehen. Die Figuren 8 und 9 zeigen einen Fluidsammelbehälter 5', der an die in der Figur 7 gezeigte Vorrichtung anschliessbar ist.

Die in den Figuren 1 bis 7 gezeigten Vorrichtungen sind insbesondere zur kombinierten Unterdruck- und Instillations-/Irrigationsbehandlung von Wunden am menschlichen oder tierischen Körper geeignet. Dementsprechend beziehen sich die nachfolgenden Erläuterungen jeweils auf die Verwendung der Vorrichtungen in der kombinierten Unterdruck- und Instillations-/Irrigationsbehandlung. Es wäre grundsätzlich aber auch möglich, diese Vorrichtungen, bei entsprechend angepasster Auslegung, für die Katheterspülung, die Augenchirurgie, die Fettabsaugung oder eine andere medizinische Anwendung zu verwenden.

Elemente mit einer identischen oder ähnlichen technischen Funktion und Wirkung sind in den Figuren 1 bis 9 bei den verschiedenen Ausführungsformen jeweils mit denselben Bezugszeichen versehen oder weisen dasselbe, jedoch mit einem Strich (`) versehene Bezugszeichen auf.

Wie es in der Figur 1 gut erkennbar ist, weist die Vorrichtung der ersten erfindungsgemässen Ausführungsform der Figuren 1 bis 4 ein Pumpaggregatgehäuse 1 mit einem daran anschliessbaren Fluidsammelbehälter 5 auf.

Das Pumpaggregatgehäuse 1 weist eine insgesamt im Wesentlichen quaderförmige Gestalt auf. In den Figuren 1 und 2 sind jeweils eine obere Wand 14, eine Vorderwand 10 sowie eine Seitenwand 12 des Pumpaggregatgehäuses 1 erkennbar. Die Seitenwand 12 weist in ihrem Eckbereich, wo sie mit der Vorderwand 10 verbunden ist, eine Einbuchtung 120 auf, welche sich entlang der gesamten Höhe der Seitenwand 12 erstreckt.

Der Fluidsammelbehälter 5 weist ebenfalls eine insgesamt im Wesentlichen quaderförmige Gestalt auf. In den Figuren 1 und 2 sind jeweils eine obere Wand 54 sowie eine Vorderwand 50 zu erkennen. Wenn der Fluidsammelbehälter 5 bestimmungsgemäss am Pumpaggregatgehäuse 1 angeschlossen ist, wie es in den Figuren 1 und 2 gezeigt ist, bildet er zusammen mit diesem eine insgesamt im Wesentlichen quaderförmige Form mit abgerundeten Aussenkanten und -ecken. Die Aussenwände des Pumpaggregatgehäuses 1 und des Fluidsammelbehälters 5 sind dabei jeweils fluchtend zueinander angeordnet.

Durch die obere Wand 54 hindurch führen zwei Schläuche aus dem Fluidsammelbehälter 5 heraus, von denen einer eine Sekretleitung S und der andere eine Hilfsleitung H bildet. Die Sekretleitung S dient dazu, den Fluidsammelbehälter 5 mit einer Kavität oder Wunde eines Patienten zu verbinden, aus welcher Körperfluide abgesaugt werden sollen, wobei die abgesaugten Körperfluide im Fluidsammelbehälter 5 gesammelt werden. Mittels der Hilfsleitung H ist es möglich, bei Bedarf die Sekretleitung S zu spülen und/oder den Druck oder die Durchflussmenge in der Sekretleitung S zu messen. Die Hilfsleitung H mündet hierzu bevorzugt in der Nähe der Kavität bzw. Wunde in die Sekretleitung S.

Die Sekretleitung S mündet mit ihrem patientenfernen Ende in das Innere des Fluidsammelbehälters 5. In seiner dem Pumpaggregatgehäuse 1 zugewandten Seitenwand weist der Fluidsammelbehälter 5 einen in den Figuren nicht erkennbaren Vakuumanschluss auf, welcher eine im Pumpaggregatgehäuse 1 angeordnete Vakuumpumpe mit dem Inneren des Fluidsammelbehälters 5 verbindet, so dass mittels der Vakuumpumpe im Inneren des Fluidsammelbehälters 5 ein Vakuum erzeugbar ist. Die Hilfsleitung H führt via den Fluidsammelbehälter 5 und einen in derselben Seitenwand wie der Vakuumanschluss angeordneten und in den Figuren ebenfalls nicht erkennbaren Hilfsanschluss direkt in das Pumpaggregatgehäuse 1.

Die Seitenwand 12 des Pumpaggregatgehäuses 1 weist eine zentral angeordnete Vertiefung 121 auf. Die Vertiefung 121 hat die Gestalt einer Halbkreisfläche und ist zur Einbuchtung 120 hin offen ausgebildet. Die Vertiefung 121 weist dieselbe Tiefe wie die Einbuchtung 120 auf. Die Seitenwand 12 bildet ausserdem einen ersten Führungskanal 122 sowie einen zweiten Führungskanal 123 in Form von geradlinig und parallel zueinander angeordneten Vertiefungen. Die beiden Führungskanäle 123 münden am obersten bzw. am untersten Punkt der halbkreisflächenförmigen Vertiefung 121 jeweils tangential in diese hinein.

Auf einen am unteren Bereich der Einbuchtung 120 der Seitenwand 12 vorgesehenen Aufsteckzapfen 16 ist ein Aufsteckteil 4 aufsetzbar, welches ungefähr dieselben Dimensionen wie die Einbuchtung 120 aufweist. Zur Aufnahme des Aufsteckzapfens 16 weist das Aufsteckteil 4 eine dazu komplementär ausgebildete Aussparung 40 auf. Der Aufsteckzapfen 16 kann insbesondere zum Einrasten in die Aussparung 40 ausgebildet sein. Weitere und/oder anders ausgebildete Befestigungsstrukturen zum Anbringen des Aufsteckteils 4 an das Pumpaggregatgehäuse 1 sind selbstverständlich denkbar.

Das Aufsteckteil 4 weist eine seitlich zur Vertiefung 121 hin offene Ausnehmung 41 auf, welche die Gestalt einer Halbkreisfläche hat. Zusammen mit der Vertiefung 121 bildet die Ausnehmung 41, wenn das Aufsteckteil 4 bestimmungsgemäss am Pumpaggregatgehäuse 1 angebracht ist, eine kreisflächenförmige Vertiefung. Innerhalb dieser kreisflächenförmigen Vertiefung ist ein Pumpenkopf 30 einer Peristaltikpumpe 3 angeordnet.

An der Peripherie des kreisförmigen Pumpenkopfes 3 sind in regelmässigen Abständen zueinander entlang der Umfangsrichtung mehrere Anpressrollen 303 frei drehbar angebracht. Der halbkreisbogenförmige Raum zwischen dem Pumpenkopf 3 und dem Aufsteckteil 4 bildet ein Schlauchbett 42. Das obere Ende dieses Schlauchbettes 42 mündet in den ersten Führungskanal 122 und das untere Ende in den zweiten Führungskanal 123. Mittels Abnehmen des Aufsteckteils 4 vom Pumpaggregatgehäuse 5 kann einfach ein Schlauch in die Führungskanäle 122 und 123 sowie das Schlauchbett 42 eingelegt bzw. daraus entnommen werden.

Im Betrieb der Vorrichtung dreht sich der Pumpenkopf 3 um seine Längsmittelachse, so dass die Anpressrollen 303 auf einem in das Schlauchbett 42 eingelegten Schlauch abrollen. Dieser Schlauch bildet eine Instillationsleitung I und ist, wie es in der Figur 1 gezeigt ist, in den ersten und den zweiten Führungskanal 122, 123 sowie das Schlauchbett 42 eingelegt. Im Bereich des Schlauchbetts 42 wird die Instillationsleitung I um 180° umgelenkt. Beim Abrollen pressen die Anpressrollen 303 den Schlauch jeweils gegen das Aufsteckteil 4, so dass eine in der Instillationsleitung I enthaltene fluide Substanz aufgrund der mechanischen Verformung des Schlauches durch diesen hindurchgedrückt und zum Wundbereich hin befördert wird. Die Peristaltikpumpe 3 wird somit insbesondere durch den Pumpenkopf 30, das Schlauchbett 42 und den entsprechend darin eingelegten Abschnitt der Instillationsleitung I gebildet.

Mit Hilfe der Peristaltikpumpe 3 wird durch die Instillationsleitung I hindurch eine fluide Substanz der Kavität oder Wunde des Patienten zugeführt. Bei der Substanz kann es sich beispielsweise um eine physiologische oder nicht physiologische Kochsalzlösung, ein Arzneimittel oder um ein Gemisch davon handeln. Die Instillationssubstanz kann zum Spülen einer Wunde oder Kavität dienen. Sie kann aber auch zum Einbringen eines Medikaments oder zur lokalen Betäubung des Wundbereichs dienen.

Die Instillationsleitung I kann insbesondere, wie es in der Figur 2 gezeigt ist, mit einem Flüssigkeitsbehälter 6 verbunden sein, in welchem eine Instillationsflüssigkeit gelagert ist. Der Flüssigkeitsbehälter 6, welcher hier als Beutel ausgebildet ist, weist einen Aufhänger 60 auf, um ihn zum Beispiel an einem Infusionsständer bzgl. der Schwerkraftrichtung oberhalb der Peristaltikpumpe 3 aufzuhängen. Auf diese Weise ist sichergestellt, dass jederzeit Instillationsflüssigkeit im Bereich der Peristaltikpumpe vorhanden ist.

Im Bereich der oberen Wand 14 des Pumpaggregatgehäuses 1 ist ein Anzeige- und Bedienfeld 20 angeordnet. Mit Hilfe des Anzeige- und Bedienfeldes 20 kann die Vorrichtung bedient, und es können insbesondere die Funktionen der Peristaltikpumpe 3 sowie der im Pumpaggregatgehäuse 1 untergebrachten Vakuumpumpe eingestellt werden. Ausserdem kann das Anzeige- und Bedienfeld 20 zum Anzeigen von Informationen zum Status der Vorrichtung, wie insbesondere aktuelle Pumpleistungen und -zyklen etc., dienen.

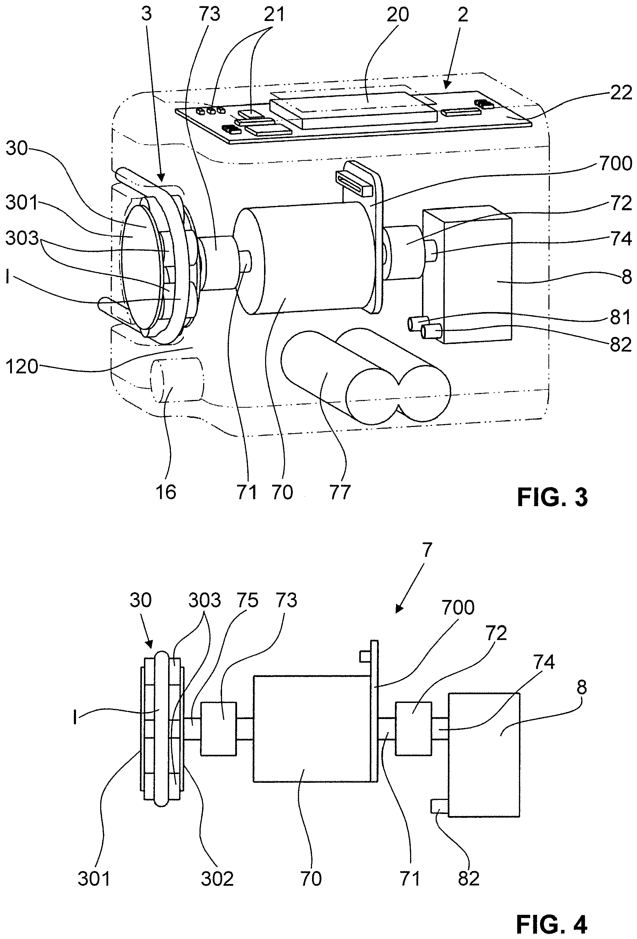

Das Innenleben des Pumpaggregatgehäuses 1 ist schematisch in den Figuren 3 und 4 gezeigt. Im Inneren des Pumpaggregatgehäuses 1 ist insbesondere ein Antriebsstrang 7 mit einem Antrieb in Form eines Motors 70 untergebracht. Der Motor 70, bei welchem es sich bevorzugt um einen bürstenlosen Gleichstrommotor handelt, dient zum Antreiben der Peristaltikpumpe 3 sowie der Vakuumpumpe, welche hier als Membranpumpe 8 ausgebildet ist. Mittels einer Befestigungsplatte 700 ist der Stator des Motors 70 drehfest am Pumpaggregatgehäuse 1 befestigt.

Der Motor 70 weist einen Rotor auf, der drehfest mit einer Motorwelle 71 verbunden ist. Aufgrund der Drehrichtung des Rotors des Motors 70 ist eine Rotationsachse des Antriebsstrangs 7 definiert. Die Motorwelle 71, deren Längsmittelachse mit der Rotationsachse zusammenfällt, ragt mit ihren beiden Endbereichen zu beiden Seiten hin aus dem Motor 70 heraus. Mittels eines ersten Freilaufs 72 ist der erste Endbereich der Motorwelle 71 mit einer ersten Antriebswelle 74 verbunden, und mittels eines zweiten Freilaufs 73 ist der zweite Endbereich der Motorwelle mit einer zweiten Antriebswelle 75 verbunden. Die Freiläufe 72 und 73 stellen Kupplungen dar, welche nur in einer Drehrichtung wirken, d.h. die Rotationsbewegung der Motorwelle 71 nur dann auf die erste bzw. zweite Antriebswelle 74, 75 übertragen, wenn sich die Motorwelle 71 in eine bestimmte Richtung dreht. Wenn die Drehgeschwindigkeit der Antriebswelle 74 bzw. 75 dabei aber bereits grösser ist als diejenige der Motorwelle 71 findet im entsprechenden Freilauf 72 bzw. 73 keine Drehmomentübertragung statt. Die Freiläufe 72 und 73 können daher auch als Überholkupplungen bezeichnet werden.

Die erste Antriebswelle 74 und die zweite Antriebswelle 75 erstrecken sich mit ihren jeweiligen Längsmittelachsen entlang der Rotationsachse des Antriebsstranges 7. Die Drehrichtungen, in welche eine Drehmomentübertragung von der Motorwelle auf die erste bzw. zweite Antriebswelle 74, 75 stattfindet, sind für die beiden Freiläufe 72 und 73 in umgekehrte Richtungen orientiert. Bei einer Drehung der Motorwelle 71 im Gegenuhrzeigersinn erfolgt deshalb nur eine Drehmomentübertragung auf die erste Antriebswelle 74, während die zweite Antriebswelle 75 stillsteht. Es wird somit in diesem Fall nur die Membranpumpe 8, nicht jedoch die Peristaltikpumpe 3 angetrieben. Bei einer Umkehrung der Drehrichtung des Motors 70 und somit der Motorwelle 71 wird hingegen nur die Peristaltikpumpe 3, nicht aber die Membranpumpe 8 angetrieben.

Der Antriebsstrang 7 wird im vorliegenden Ausführungsbeispiel somit durch den Motor 70, die Motorwelle 71, die beiden Freiläufe 72 und 73 sowie durch die erste Antriebswelle 74 und die zweite Antriebswelle 75 gebildet. Aufgrund der beiden in umgekehrte Richtungen orientierten Freiläufe 72 und 73 ist bei dieser Ausführungsform sichergestellt, dass in jedem Fall nur eine der beiden Pumpen 3 und 8 in Betrieb ist. Je nach Drehrichtung des Motors 70 wird entweder die Peristaltikpumpe 3 oder die Membranpumpe 8 angetrieben.

Die Membranpumpe 8 dient dazu, im Fluidsammelbehälter 5 ein Vakuum zu generieren, um via die Sekretleitung S Körperfluide anzusaugen und im Fluidsammelbehälter zu sammeln. Dazu weist die Membranpumpe 8 einen Vakuumanschluss 81 auf, welcher via eine in den Figuren nicht gezeichnete Leitung mit dem behälterseitigen Vakuumanschluss des Fluidsammelbehälters 5 verbindbar ist. Ausserdem weist die Membranpumpe 8 einen Auslass 82 auf, um durch diesen hindurch die angesaugte Luft auszustossen. Der Auslass 82 ist hierzu mit einer in den Figuren nicht gezeigten Leitung verbunden, die in die Umgebung, d.h. nach aussen hin, mündet. Der Antrieb der Membranpumpe 8 erfolgt über die erste Antriebswelle 74.

Die Peristaltikpumpe 3 weist, wie es bereits schon erwähnt wurde, mehrere Anpressrollen 303 auf. Diese Anpressrollen 303 sind um ihre jeweiligen Achsen frei drehbar an einem Pumpenkopf 30 gelagert und zwischen zwei sich parallel zueinander erstreckenden, kreisrunden Platten 301 und 302 angeordnet. Die Achsen der Anpressrollen 303 verbinden dabei jeweils die beiden Platten 301 und 302 miteinander. Der Pumpenkopf 30 ist über die zweite Antriebswelle 75 in eine Drehbewegung um die Rotationsachse des Antriebsstranges 7 versetzbar.

Im Inneren des Pumpaggregatgehäuses 1 ist ausserdem eine Elektronikeinheit 2 untergebracht. Die Elektronikeinheit 2 weist eine Leiterplatine (engl. printed circuit board, PCB) 22 auf, auf welcher Elektronikkomponenten 21, wie insbesondere eine Steuereinheit, eine Motorendstufe, Druckmesssensorik, ein Speicherchip etc., angeordnet sind. Auf dem PCB 22 sind ausserdem ein Display und Bedienelemente, allenfalls ein Touchscreen, angeordnet, um das Anzeige- und Bedienfeld 20 zu bilden. Die Elektronikeinheit 2 dient insbesondere zum Einstellen und Regeln der Drehrichtung und der Drehgeschwindigkeit des Motors 70, in Abhängigkeit der vom Benutzer am Anzeige- und Bedienfeld 20 gemachten Eingaben.

Zur Versorgung des Motors 70 sowie der Elektronikeinheit 2 mit elektrischer Energie ist ausserdem ein Energiespeicher in Form eines Akkumulators 77 im Inneren des Pumpaggregatgehäuses 1 untergebracht.

Die in den Figuren 5 und 6 gezeigte zweite erfindungsgemässe Ausführungsform ist bis auf einige wenige Unterschiede, welche nachfolgend erläutert werden, gleich ausgebildet wie die Ausführungsform der Figuren 1 bis 4.

Im Gegensatz zur Ausführungsform der Figuren 1 bis 4 weist diejenige der Figuren 5 und 6 nur einen einzigen Freilauf 73 auf, der die Motorwelle 71 mit derjenigen Antriebswelle 75 verbindet, welche den Pumpenkopf 30 antreibt. Der Antrieb der Membranpumpe 8 erfolgt direkt via die Motowelle 71. Es ist dadurch möglich, die beiden Pumpen 3 und 8 gleichzeitig anzutreiben, so dass zeitgleich eine Sekretabsaugung durch die Sekretleitung S als auch eine Zuführung von Instillationsflüssigkeit durch die Instillationsleitung I erfolgen kann. Bei einer Umkehrung der Drehrichtung des Motors 70 wird aufgrund des Freilaufs 73 aber nur die Membranpumpe 8 angetrieben.

Um beim zeitgleichen Betrieb der Membranpumpe 8 und der Peristaltikpumpe 3 aber dennoch eine beliebige Einstellung des im Fluidsammelbehälter 5 und in der Sekretleitung S herrschenden Vakuums zu ermöglichen, ist bei der vorliegenden Ausführungsform via eine Verbindungsleitung 93 ein Pneumatikventil 9 am Vakuumanschluss 81 der Membranpumpe 8 angeschlossen. Das Pneumatikventil 9 weist einen Vakuumanschluss 91 auf, welcher via eine in den Figuren nicht gezeichnete Leitung mit dem behälterseitigen Vakuumanschluss des Fluidsammelbehälters 5 verbunden ist. Ausserdem weist das Pneumatikventil einen Einlass 92 auf, welcher via eine weitere, ebenfalls nicht gezeigte Leitung, in die Umgebung mündet.

Je nach Ventilstellung des Pneumatikventils 9 liegt am Vakuumanschluss 91 die gesamte von der Membranpumpe 8 erzeugte Saugleistung an oder nur ein Teil davon. Das Pneumatikventil 9 kann sogar derart eingestellt sein, dass die Verbindungsleitung 93 direkt mit dem Einlass 92 verbunden ist, so dass im Fluidsammelbehälter 5 und somit in der Sekretleitung S trotz des Betriebs der Membranpumpe 8 Atmosphärendruck herrscht. Bei einer derartigen Ventilstellung kann Instillationsflüssigkeit durch die Instillationsleitung I zugeführt werden, ohne dass gleichzeitig eine Absaugung durch die Sekretleitung S erfolgt. Die Stellung des Pneumatikventils 9 ist bevorzugt von der Elektronikeinheit 2, in Abhängigkeit der vom Benutzer am Anzeige- und Bedienfeld 20 gemachten Eingaben, regelbar.

Eine dritte, nicht erfindungsgemässe Ausführungsform ist in den Figuren 7 bis 9 gezeigt. Die Vorrichtung dieser Ausführungsform weist ein in der Figur 7 gezeigtes Pumpaggregatgehäuse 1' auf, an welches ein in den Figuren 8 und 9 gezeigter Fluidsammelbehälter 5' anschliessbar ist.

Das Pumpaggregatgehäuse 1' hat auch hier eine insgesamt im Wesentlichen quaderförmige Gestalt mit einer in der Figur 7 nicht gezeigten Vorderwand, einer Rückwand 11', einer ersten Seitenwand 12' und einer zweiten Seitenwand 13' sowie einer oberen Wand 14' und einer unteren Wand 15'. Die Vorderwand und die Rückwand 11' weisen je eine Wandkante auf, welche der dazwischen angeordneten ersten Seitenwand 12' vorstehen. Der Fluidsammelbehälter 5' ist zwischen diesen Wandkanten gehalten und dadurch einfach, aber trotzdem sicher und geschützt an das Pumpaggregatgehäuse 1' befestigbar.

Zum Einhängen und Halten des Fluidsammelbehälters 5' am Pumpaggregatgehäuse 1' sind am Pumpaggregatgehäuse 1' Aufnahmehaken 190' vorgesehen, in welche entsprechend ausgebildete und angeordnete Zapfen 554` des Fluidsammelbehälters 5' eingreifen können. Mittels einer an einem federbelasteten Element angebrachten Rückhaltenase 191', welche zum Einschnappen in eine am Fluidsammelbehälter 5' ausgebildete Rastkerbe 553' ausgebildet ist, wird der Fluidsammelbehälter 5' am Pumpaggregatgehäuse 1' gesichert. Um die Rastverbindung zwischen der Rückhaltenase 191' und der Rastkerbe 553' zu lösen, kann das federbelastete Element, an welchem die Rückhaltenase 191' angebracht ist, entgegen der Federkraft nach unten gedrückt werden.

Die vorstehenden Wandkanten der Vorderwand und der Rückwand 11' sowie die Aufnahmehaken 190' und die Rückhaltenase 191' bilden gemeinsam eine Behälteraufnahme 19` des Fluidsammelbehälters 5'.

Das Pumpaggregatgehäuse 1' weist einen gehäuseseitigen Vakuumanschluss 17' auf, welcher beim Anbringen des Fluidsammelbehälters 5' an das Pumpaggregatgehäuse 1' an einen entsprechend am Fluidsammelbehälter 5' vorgesehenen behälterseitigen Vakuumanschluss 551' gekoppelt wird, so dass via die Anschlüsse 17` und 551' im Inneren des Fluidsammelbehälters 5' ein Vakuum erzeugbar ist.

Innerhalb der ersten Seitenwand 12' ist ausserdem eine Adapteraufnahme 18' vorgesehen, welche zur Aufnahme eines in den Figuren nicht gezeigten Schlauchadapters dient. Der Schlauchadapter verbindet eine in den Figuren nicht gezeigte Sekretleitung via einen am Fluidsammelbehälter 5' vorgesehenen behälterseitigen Sekretanschluss 552' mit dem Inneren des Fluidsammelbehälters 5'.

Im Inneren des Pumpaggregatgehäuses 1' ist ein Antriebsstrang 7' mit einem Motor 70` und einer mit dem Motor 70' verbundenen Motorwelle 71' untergebracht. Beim Motor 70' kann es sich auch hier insbesondere um einen bürstenlosen Gleichstrommotor handeln.

Die Motorwelle 71' treibt mit einem ersten Endbereich direkt eine in der Figur 7 aus darstellerischen Gründen nicht gezeigte Membranpumpe an. Mit einem zweiten Endbereich, welcher in der Figur 7 nicht sichtbar ist, ist die Motorwelle 71' mit einem Freilauf 72' verbunden, welcher die Motorwelle 71' mit einer Antriebswelle 75` verbindet. Die Antriebswelle 75`, welche sich entlang der Rotationsachse der Motorwelle 71' erstreckt, ragt durch die erste Seitenwand 12` hindurch.

Auf der Aussenseite des Pumpaggregatgehäuses 1' ist ein Kopplungselement 76` drehfest am Ende der Antriebswelle 75' angebracht. Das Kopplungselement 76' hat die Form eines Zahnrades. Im vorliegenden Fall ist es ein Zahnrad mit vier Zähnen. Via das Kopplungselement 76' kann ein im Fluidsammelbehälter 5` integrierter Pumpenkopf 30' einer Peristaltikpumpe 3' angetrieben werden, wenn der Fluidsammelbehälter 5' bestimmungsgemäss am Pumpaggregatgehäuse 1' angebracht ist.

Grundsätzlich ist es natürlich auch denkbar, dass der Pumpenkopf 30' nicht in einem Fluidsammelbehälter integriert ist, sondern an einem beliebigen anderen Teil, wie beispielsweise einem Zwischenteil, welches zwischen dem Pumpaggregatgehäuse 1' und einem Fluidsammelbehälter angekoppelt werden kann. Der Pumpenkopf 30' könnte insbesondere auch einen Teil eines Behälters darstellen, in welchem die Instillationssubstanz aufgenommen ist. Der Behälter könnte ein Identifikationsmerkmal aufweisen und die Vorrichtung eine Identifikationseinheit, um zu identifizieren, welche Art einer Substanz im Behälter enthalten ist. Die Vorrichtung könnte dann dazu ausgebildet sein, in Abhängigkeit der identifizierten Art der Substanz einen von mehreren möglichen Betriebsmodi zum Antreiben der ersten Pumpe und der zweiten Pumpe bzw. des Kopplungselements auszuwählen oder die Auswahl der Betriebsmodi für den Benutzer in Abhängigkeit der Substanz zu beschränken. Das Identifikationsmerkmal kann insbesondere elektronisch auslesbar sein, zum Beispiel mittels RFID. Eine derartige Identifikation und anschliessende Vorauswahl von Betriebsmodi ist selbstverständlich auch dann denkbar, wenn der Pumpenkopf nicht im Behälter mit der zu identifizierenden Substanz integriert ist.

Der Fluidsammelbehälter 5' weist eine Vorderwand 50`, eine in den Figuren nicht sichtbare Rückwand, zwei Seitenwände 52` und 53' sowie eine obere Wand 54 und eine ebenfalls nicht sichtbare untere Wand auf. Diese Wände werden durch ein aus einem opaken Material hergestellten Basisteil 55` und einem transparenten Teil 56' gebildet. An derjenigen Seitenwand 52`, welche ausschliesslich durch das transparente Teil 56` gebildet wird, ist eine Füllstandsskala 560' vorgesehen.

Diejenige Seitenwand 53', welche ausschliesslich durch das Basisteil 55' gebildet wird, weist eine zentral angeordnete, ringförmige Vertiefung 555` auf. Innerhalb der ringförmigen Vertiefung 555` bildet die Seitenwand 53' einen konzentrisch angeordneten Lagerzapfen 556`, der eine obere, nach oben hin offene Aussparung 557` aufweist.

In der ringförmigen Vertiefung 555` ist ein Hohlrad 57` aufgenommen, welches frei drehbar um den Lagerzapfen 556' herum angeordnet ist. Im Hohlrad 57' sind in den Figuren nicht sichtbare Anpressrollen in regelmässigen Abständen entlang der Umfangsrichtung frei drehbar angebracht. Die Anpressrollen dienen beim Drehen des Hohlrades 57' zum Abrollen auf einen in der ringförmigen Vertiefung 555` auf der radialen Aussenseite des Hohlrades 57` eingelegten Schlauch. Der Schlauch kann insbesondere eine Instillationsleitung I bilden. Indem die Anpressrollen auf dem Schlauch abrollen, wird dieser zusammengepresst und ein sich darin befindliches Fluid befördert.

Auf seiner radialen Innenseite ist am Hohlrad 57` eine umlaufende Verzahnung 570` ausgebildet. Die Verzahnung 570' gerät beim bestimmungsgemässen Anbringen des Fluidsammelbehälters 5' an das Pumpaggregatgehäuse 1' in Eingriff mit dem Kopplungselement 76', so dass bei Drehen der Antriebswelle 75' die Drehbewegung via das Kopplungselement 76' auf das Hohlrad 57` übertragen wird.

Das Hohlrad 57` bildet somit zusammen mit der ringförmigen Vertiefung 555` und der darin eingelegten Instillationsleitung I eine Peristaltikpumpe 3`, wobei das Hohlrad 57` den Pumpenkopf 30' bildet. Um die Instillationsleitung I zur ringförmigen Vertiefung 555' hin und wieder von dieser weg zu führen, sind innerhalb der ersten Seitenwand 12' entsprechende Führungskanäle zur Aufnahme der Instillationsleitung I ausgebildet, welche geradlinig und parallel zueinander von der ringförmigen Vertiefung 555' nach oben bis zur oberen Wand 54' verlaufen.

Vorteilhaft werden im Wesentlichen sämtliche Teile des Fluidsammelbehälters 5', inklusive dem Hohlrad 57` und den daran angebrachten Anpressrollen, im Spritzgussverfahren hergestellt. Da der Fluidsammelbehälter 5' üblicherweise ein Wegwerfteil darstellt, welches oft bereits nach einer einzigen Anwendung ausgetauscht und entsorgt wird, sind die Anforderungen an den darin integrierten Pumpenkopf 30 verhältnismässig gering. Die Herstellungskosten für die gesamte Vorrichtung können dadurch erheblich gesenkt werden im Vergleich zu einer Vorrichtung, bei welcher der Pumpenkopf im oder am Pumpaggregatgehäuse angeordnet ist und dadurch für eine viel längere Lebensdauer ausgelegt sein muss.

Bei allen Ausführungsformen kann der Antriebsstrang 7 bzw. 7` ausserdem eines oder mehrere Getriebe aufweisen, um die Drehgeschwindigkeit des Pumpenkopfes 30 und/oder die Pumpfrequenz der Membranpumpe 8 gegenüber derjenigen des Motors 70 zu verändern. Mittels des zumindest einen Getriebes können die Peristaltikpumpe 3, 3' und die Membranpumpe 8, obwohl sie einen gemeinsamen Antrieb in Form des Motors 70 bzw. 70` haben, beliebig mit unterschiedlichen oder gleichen Pumpfrequenzen betrieben werden. Das oder die Getriebe können, müssen aber nicht, mit den Freiläufen 72, 72` und/oder 73 kombiniert werde. Sie können somit zusätzlich oder alternativ zu den Freiläufen 72, 72` und 73 vorgesehen sein.

Selbstverständlich ist die hier beschriebene Erfindung nicht auf die erwähnten Ausführungsformen beschränkt und eine Vielzahl von Abwandlungen ist möglich. So müssen die erste und die zweite Pumpe zum Beispiel nicht zwingend in Form einer Membranpumpe und einer Peristaltikpumpe vorliegen, sondern es können, je nach Anwendung, für beide Pumpen grundsätzlich beliebige Pumpenarten eingesetzt werden. Die erwähnten Ausführungsformen betreffen bevorzugt Vorrichtungen, welchen jeweils tragbar ausgebildet sind, so dass sie vom Benutzer alleine und ohne übermässigen Kraftaufwand bequem tragbar sind. Grundsätzlich lassen sich die beschriebenen Vorrichtungen beliebig dimensionieren. Eine Vielzahl weiterer Abwandlungen ist denkbar.

**BEZUGSZEICHENLISTE**

| | | | |
|---|---|---|---|
| 1, 1' | Pumpaggregatgehäuse | 40 | Aussparung |
| 10 | Vorderwand | 41 | Ausnehmung |
| 11' | Rückwand | 42 | Schlauchbett |
| 12, 12' | Seitenwand | | |
| 120 | Einbuchtung | 5, 5' | Fluidsammelbehälter |
| 121 | Vertiefung | 50, 50' | Vorderwand |
| 122 | Erster Führungskanal | 52` | Seitenwand |
| 123 | Zweiter Führungskanal | 53' | Seitenwand |
| 13' | Seitenwand | 54, 54' | Obere Wand |
| 14, 14' | Obere Wand | 55` | Basisteil |
| 15' | Untere Wand | 551' | Behälterseitiger |
| 16 | Aufsteckzapfen | | Vakuumanschluss |
| 17' | Gehäuseseitiger Vakuumanschluss | 552` | Behälterseitiger Sekretanschluss |
| 18' | Adapteraufnahme | 553` | Rastkerbe |
| 19' | Behälteraufnahme | 554' | Zapfen |
| 190` | Aufnahmehaken | 555` | Ringförmige Vertiefung |
| 191' | Rückhaltenase | 556` | Lagerzapfen |
| | | 557` | Aussparung |
| 2 | Elektronikeinheit | 56` | Transparentes Teil |
| 20 | Anzeige- und Bedienfeld | 560` | Füllstandsskala |
| 21 | Elektronikkomponenten | 57` | Hohlrad |
| 22 | PCB | 570` | Verzahnung |
| 3, 3' | Peristaltikpumpe | 6 | Flüssigkeitsbehälter |
| 30, 30' | Pumpenkopf | 60 | Aufhänger |
| 301 | Platte | | |
| 302 | Platte | 7, 7' | Antriebsstrang |
| 303 | Anpressrollen | 70, 70` | Motor |
| | | 700 | Befestigungsplatte |
| 4 | Aufsteckteil | 71, 71' | Motorwelle |
| 72, 72` | Erster Freilauf | | |
| 73 | Zweiter Freilauf | 9 | Pneumatikventil |
| 74 | Erste Antriebswelle | 91 | Vakuumanschluss |
| 75, 75` | Zweite Antriebswelle | 92 | Einlass |
| 76` | Kopplungselement | 93 | Verbindungsleitung |
| 77 | Akkumulator | | |
| | | S | Sekretleitung |
| 8 | Membranpumpe | H | Hilfsleitung |
| 81 | Vakuumanschluss | I | Instillationsleitung |
| 82 | Auslass | | |

## Patentansprüche

1. Vorrichtung zum Absaugen von Körperfluiden und zum Zufuhren einer Substanz zu einem menschlichen oder tierischen Körper, aufweisend
eine erste Pumpe (8) zum Absaugen der Körperfluide;
eine zweite Pumpe (3), um mittels der zweiten Pumpe (3, 3') die Substanz zum Körper zu befördern; sowie
einen Antrieb (70, 70') zum Antreiben der ersten Pumpe (8);
wobei derselbe Antrieb (70, 70'), welcher zum Antreiben der ersten Pumpe (8) dient, auch zum Antreiben der zweiten Pumpe (3, 3') dient,
**dadurch gekennzeichnet, dass**
die Vorrichtung ausserdem zumindest einen ersten Freilauf (72, 73) aufweist, mittels welchem die erste Pumpe (8) oder die zweite Pumpe (3, 3') an den Antrieb (70, 70') gekoppelt ist, wobei es sich bei der ersten Pumpe (8) um eine Vakuumpumpe handelt, und wobei die Vorrichtung zusätzlich ein Ventil aufweist, mittels welchem die Vakuumpumpe mit der Umgebung verbindbar ist, um zumindest teilweise anstatt Körperfluide Luft aus der Umgebung anzusaugen.

2. Vorrichtung nach Anspruch 1 , wobei es sich bei der Vakuumpumpe um eine Membranpumpe handelt.

3. Vorrichtung nach Anspruch 1 oder 2, wobei das Ventil ein Pneumatikventil (9) ist.

4. Vorrichtung nach einem der vorhergehenden Ansprüche, wobei es sich bei der zweiten Pumpe um eine Peristaltikpumpe (3, 3') handelt.

5. Vorrichtung nach einem der vorhergehenden Ansprüche, wobei die Vorrichtung zur kombinierten Unterdruck- und Instillationsbehandlung von Wunden am menschlichen oder tierischen Körper dient.

6. Vorrichtung nach einem der vorhergehenden Ansprüche, wobei die Vorrichtung zur kombinierten Absaugung und Spülung bei der Liposuktion oder zur kombinierten Absaugung und Spülung bei der Augenchirurgie dient.

7. Vorrichtung nach einem der vorhergehenden Ansprüche, wobei mittels des ersten Freilaufs (72) die erste Pumpe (8) an den Antrieb (70, 70') gekoppelt ist, und wobei zudem ein zweiter Freilauf (73) vorhanden ist, welcher eine im Vergleich zum ersten Freilauf (72) entgegengesetzte Freilaufrichtung aufweist, und mittels welchem die zweite Pumpe (3, 3') an den Antrieb (70, 70') gekoppelt ist.

8. Vorrichtung nach einem der vorhergehenden Ansprüche, wobei es sich beim Antrieb um einen bürstenlosen Gleichstrommotor (70, 70') handelt.

9. Vorrichtung nach einem der vorhergehenden Ansprüche, aufweisend ein Pumpaggregatgehäuse (1, ) mit einem Innenraum, in welchem zumindest die erste Pumpe (8) und der Antrieb (70, 70') untergebracht sind.

10. Vorrichtung nach Anspruch 9, wobei die zweite Pumpe (3) auf einer Aussenseite des Pumpaggregatgehäuses (1, 1') angeordnet ist.

11. Vorrichtung nach einem der vorhergehenden Ansprüche, aufweisend einen Antriebsstrang (7, 7'), bei welchem der Antrieb (70, 70') zwischen der ersten Pumpe (8) und der zweiten Pumpe (3, 3') angeordnet ist.

12. Vorrichtung nach einem der vorhergehenden Ansprüche, wobei die Vorrichtung derart ausgebildet ist, dass die Pumpleistung der ersten Pumpe (8) einerseits und die Pumpleistung der zweiten Pumpe (3, 3') andererseits weitgehend unabhängig voneinander einstellbar sind.

13. Vorrichtung nach einem der vorhergehenden Ansprüche, wobei ein Behälter mit an die Vorrichtung anbringbar ist, der die zuzuführende Substanz beinhaltet, und wobei der Behälter ein Identifikationsmerkmal und die Vorrichtung eine Identifikationseinheit aufweist, um zu identifizieren, welche Art einer Substanz im Behälter enthalten ist, und wobei die Vorrichtung dazu ausgebildet ist, in Abhängigkeit der identifizierten Art der Substanz einen von mehreren möglichen Betriebsmodi zum Antreiben der ersten Pumpe (8) und der zweiten Pumpe (3, 3') auszuwählen.

## Claims

1. A device for aspirating body fluids and supplying a substance to a human or animal body, comprising
a first pump (8) for aspiration of the body fluids;
a second pump (3) for conveying the substance to the body by means of the second pump (3, 3');
and
a drive (70, 70') for driving the first pump (8);
wherein the same drive (70, 70') which serves to drive the first pump (8) also serves to drive the second pump (3, 3'),
**characterized in that**
the device further comprises at least a first freewheel (72, 73), by means of which the first pump (8) or the second pump (3, 3') is coupled to the drive (70, 70'), wherein the first pump (8) is a vacuum pump, and wherein the device additionally comprises a valve, which allows the vacuum pump to be connected to the environment in order to aspirate air from the environment at least partially instead of body fluids.

2. The device according to claim 1, wherein the vacuum pump is a diaphragm pump.

3. The device according to claims 1 or 2, wherein the valve is a pneumatic valve (9).

4. The device according to any one of the preceding claims, wherein the second pump is a peristaltic pump (3, 3').

5. The device according to any one of the preceding claims, wherein the device is for combined suppression and instillation treatment of wounds on the human or animal body.

6. The device according to any one of the preceding claims, wherein the device is for combined aspiration and irrigation in liposuction or for combined aspiration and irrigation in eye surgery.

7. The device according to any one of the preceding claims, wherein the first pump (8) is coupled to the drive (70, 70') by means of the first freewheel (72), and wherein furthermore a second freewheel (73) is present, which has an opposite freewheeling direction compared to the first freewheel (72), and by means of which the second pump (3, 3') is coupled to the drive (70, 70').

8. The device according to any one of the preceding claims, wherein the drive is a brushless DC motor (70, 70').

9. The device according to any one of the preceding claims, comprising a pump unit housing (1) having an interior in which at least the first pump (8) and the drive (70, 70') are housed.

10. The device according to claim 9, wherein the second pump (3) is arranged on an exterior side of the pump unit housing (1, 1').

11. The device according to any one of the preceding claims, comprising a drive train (7, 7'), wherein the drive (70, 70') is arranged between the first pump (8) and the second pump (3, 3').

12. The device according to any one of the preceding claims, wherein the device is designed in such a way that the pumping power of the first pump (8), on the one hand, and the pumping power of the second pump (3, 3') on the other hand are adjustable largely independently of one another.

13. The device according to any one of the preceding claims, wherein a container containing the substance to be supplied is attachable to the device, and wherein the container has an identification feature and the device has an identification unit for identifying which type of substance is contained in the container, and wherein the device is configured to select, in dependence of the identified type of substance, one of several possible operating modes for driving the first pump (8) and the second pump (3, 3').

## Revendications

1. Dispositif pour aspirer des fluides corporels et délivrer une substance à un corps humain ou animal, comprenant
une première pompe (8) pour aspirer les fluides corporels ;
une deuxième pompe (3) pour délivrer la substance au corps au moyen de la deuxième pompe (3, 3') ; et
un entraînement (70, 70') pour entraîner la première pompe (8) ;
dans lequel le même entraînement (70, 70') qui est utilisé pour entraîner la première pompe (8) est également utilisé pour entraîner la deuxième pompe (3, 3'),
**caractérisé en ce que**
le dispositif comporte en outre au moins une première roue libre (72, 73) au moyen de laquelle la première pompe (8) ou la deuxième pompe (3, 3') est couplée à l'entraînement (70, 70'), dans lequel la première pompe (8) est une pompe à vide, et dans lequel le dispositif comporte en outre une vanne au moyen de laquelle la pompe à vide peut être connectée à l'environnement afin d'aspirer au moins partiellement de l'air de l'environnement au lieu de fluides corporels.

2. Dispositif selon la revendication 1, dans lequel la pompe à vide est une pompe à membrane.

3. Dispositif selon la revendication 1 ou 2, dans lequel la vanne est une vanne pneumatique (9).

4. Dispositif selon l'une des revendications précédentes, dans lequel la deuxième pompe est une pompe péristaltique (3, 3').

5. Dispositif selon l'une des revendications précédentes, dans lequel le dispositif est utilisé pour le traitement combiné par pression négative et instillation de plaies sur le corps humain ou animal.

6. Dispositif selon l'une des revendications précédentes, dans lequel le dispositif est utilisé pour l'aspiration et le rinçage combinés en liposuccion ou pour l'aspiration et le rinçage combinés en chirurgie oculaire.

7. Dispositif selon l'une des revendications précédentes, dans lequel la première pompe (8) est couplée à l'entraînement (70, 70') au moyen de la première roue libre (72), et dans lequel est en outre présente une deuxième roue libre (73) dont le sens de rotation en roue libre est opposé à celui de la première roue libre (72), et au moyen de laquelle la deuxième pompe (3, 3') est couplée à l'entraînement (70, 70').

8. Dispositif selon l'une des revendications précédentes, dans lequel l'entraînement est un moteur à courant continu sans balais (70, 70').

9. Dispositif selon l'une des revendications précédentes, comportant un carter de pompe (1, 1') avec une chambre intérieure dans laquelle sont logés au moins la première pompe (8) et l'entraînement (70, 70').

10. Dispositif selon la revendication 9, dans lequel la deuxième pompe (3) est agencée sur un côté externe du carter de pompe (1, 1').

11. Dispositif selon l'une des revendications précédentes, comportant un groupe d'entraînement (7, 7') dans lequel l'entraînement (70, 70') est agencé entre la première pompe (8) et la deuxième pompe (3, 3').

12. Dispositif selon l'une des revendications précédentes, dans lequel le dispositif est conçu de telle sorte que la capacité de pompage de la première pompe (8) d'une part et la capacité de pompage de la deuxième pompe (3, 3') d'autre part peuvent être ajustées en grande partie indépendamment l'une de l'autre.

13. Dispositif selon l'une des revendications précédentes, dans lequel un réservoir contenant la substance à délivrer peut être attaché au dispositif, et dans lequel le réservoir présente une caractéristique d'identification et le dispositif comporte une unité d'identification pour identifier le type de la substance contenue dans le récipient, et dans lequel le dispositif est agencé pour sélectionner un mode parmi plusieurs modes de fonctionnement possibles pour entraîner la première pompe (8) et la deuxième pompe (3, 3') en fonction du type de substance identifié.
